(19) Europäisches Patentamt
European Patent Office
Office européen des brevets

(11) Publication number: 0 167 153
B1

(12) EUROPEAN PATENT SPECIFICATION

(45) Date of publication of patent specification: 22.02.89

(51) Int. Cl.$^4$: C 07 C 50/02, C 07 C 46/08

(21) Application number: 85108202.4

(22) Date of filing: 02.07.85

# (54) Process for the preparation of 2,3,5-trimethylbenzoquinone.

(30) Priority: 03.07.84 JP 137710/84

(43) Date of publication of application:
08.01.86 Bulletin 86/02

(45) Publication of the grant of the patent:
22.02.89 Bulletin 89/08

(84) Designated Contracting States:
CH DE FR LI

(56) References cited:
EP-A-0 070 665
EP-A-0 107 427
EP-A-0 127 888
US-A-4 442 036

(73) Proprietor: MITSUBISHI GAS CHEMICAL COMPANY, INC.
5-2, Marunouchi 2-chome Chiyoda-Ku
Tokyo (JP)

(72) Inventor: Isshiki, Tomiya
14-11, 2-chome Umegaoka
Setagaya-ku Tokyo (JP)
Inventor: Yui, Tomoyuki
22-1, 3-chome, Miyazono Nagareyama-shi
Chiba-ken (JP)
Inventor: Abe, Mitsuo
11-18, 5-chome Kana-machi
Katsushika-ku Tokyo (JP)
Inventor: Jono, Masahiro
17-5, 5-chome Shinjuku
Katsushika-ku Tokyo (JP)

(74) Representative: Türk, Dietmar, Dr. rer. nat. et al
Türk, Gille + Hrabal Patentanwälte Bruckner Strasse 20
D-4000 Düsseldorf 13 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 167 153 B1

Courier Press, Leamington Spa, England.

**Description**

Background of the Invention

The present invention relates to a process for the preparation of 2,3,5-trimethylbenzoquinone (hereinafter abbreviated to "TMBQ"). More particularly, it is concerned with a process for preparing TMBQ by oxidizing 2,3,6-trimethylphenol (hereinafter abbreviated to "TMP") with oxygen in an aliphatic alcohol having from 5 to 10 carbon atoms in the presence of an aqueous catalyst solution comprising a copper halogeno complex and an alkali metal halide. TMBQ is a useful substance as an intermediate product in the preparation of Vitamin E.

Various methods are known in the art for the preparation of TMBQ through oxidation of TMP in the presence of a catalyst. Japanese Patent Publication No. 17585/1978, for example, describes a method of oxidizing TMP with oxygen in the presence of copper and halogen ions. Japanese Patent Publication No. 2446/1974 discloses a method using a cobalt complex as a catalyst.

From EP—A—1199 it is known to prepare p-benzoquinone in the presence of 2,3,6-trimethylphenol with oxygen in methanol as reaction medium in the presence of a copper halogeno compound and an alkali-metal halide. 2,3,6-Trimethylphenol is used as reducing agent for the copper catalyst.

From FR—A—2 245 602 the oxidation of methylphenols to the coresponding benzoquinone in the presence of a copper-halogeno compound is known. The reaction is carried out in water and/or a water-miscible aliphatic alcohol as methanol, ethanol or isopropanol.

These known methods, however, have various disadvantages. One of the disadvantages is that the catalytic activity in a reaction system containing water is poor. Another disadvantage is that since it is essential to use organic solvents easily soluble in water, the catalyst recovery operation is complicated and, furthermore, the amount of energy to be consumed is markedly increased because a large amount of water is required to evaporate for the recovery of the catalyst. In the method of Japanese Patent Publication No. 17585/1978, satisfactory results can be obtained only when the reaction is carried out using a copper chloride catalyst and a dimethylformamide or ethylene glycol solvent. That is, when a copper bromide catalyst, for example, is used, or the reaction is carried out with a copper chloride catalyst in acetone containing water, the yield and selectivity of TMBQ are not satisfactory. This means that in the case of the catalyst comprising copper and halogen ion, satisfactory results can be obtained only when the reaction system does not contain water and copper chloride is used as the catalyst. When copper chloride is used as the catalyst, it is required in a molar amount 0.5 to 2 times as much as TMBQ.

With the known catalysts comprising copper and halogen ion, as described above, satisfactory yield and selectivity can be obtained only under limited conditions. Therefore, they are unsuitable for practical use.

In order to overcome the above problems of the prior art, the present inventors have already proposed a process using a catalyst comprising a copper halogeno complex and an alkali metal halide (U.S. Serial No. 615,125 filed May 30, 1984). This catalyst has many advantages over the known catalysts. The major advantages are: (1) the reaction can be efficiently carried out without any trouble even in a reaction system containing water; (2) the catalyst solution can be easily separated from the reaction mixture because the reaction readily proceeds in organic solvents which are less soluble in water; and (3) a gas of low oxygen concentration can be used as a reaction gas because of high catalytic activity.

However, even if the above catalyst is used, the following problems arise when the reaction is carried out batchwise. (1) Hexamethylbiphenol (hereinafter abbreviated to "HMBP") having the chemical structure:

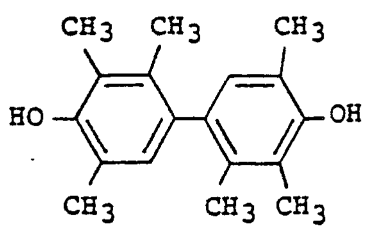

is by-produced in relatively large amounts, (2) in order to obtain TMBQ in high selectivity and high yield, it is necessary to use the copper halogeno complex as the major component of the catalyst in large amounts as much as equimolar to or more than TMP; and (3) it is difficult to control the reaction temperature because of large amount of heat generated by oxidation reaction.

Summary of the Invention

The present invention is intended to provide a process for the production of TMBQ whereby the amount of a catalyst used can be greatly reduced, the yield of TMBQ can be increased while decreasing the amount of HMBP formed, and the reaction temperature can be easily controlled, provided that an expensive oxidizing agent is not necessary to use, the product is easily separated from the reaction mixture, and the catalyst is of high activity, is not reduced in activity by water, has a long life, is easy in the recovery from the reaction mixture and in isolation, and can be used repeatedly or in a recycled manner.

Accordingly the present invention relates to a process for preparing TMBQ by contacting TMP with oxygen gas or oxygen-containing gas having an oxygen concentration of at least 20% in an aqueous solution of a catalyst comprising a copper halogeno complex represented by the following general formula (I) and an alkali metal halide,

$$M_l[Cu(II)_mX_n]_p \qquad (I)$$

wherein M is an alkali metal belonging to IA of the Periodic Table or ammonium, Cu(II) is a divalent copper, X is a halogen, l is an integer of from 1 to 3, m is 1 or 2, n is an integer of from 3 to 8, and p is 1 or 2, provided that $l + 2\,mp = np$; wherein the catalytic reaction of TMP with the oxygen gas or the oxygen-containing gas is carried out semi-batchwise while continuously adding a solution of TMP in an aliphatic alcohol having from 5 to 10 carbon atoms to the aqueous solution of the catalyst to which an aliphatic alcohol having from 5 to 10 carbon atoms, cupric hydroxide, cuprous chloride, or a mixture thereof is previously added.

Detailed Description of the Invention

The catalyst comprising a copper halogeno complex and an alkali metal halide, which is used in the present invention, is novel and is excellent in a capability to oxidize TMP with oxygen and to convert it into TMBQ in a high selectivity. That is, it has been unexpectedly found that the catalyst used in the present invention is superior in the rate of reaction and selectivity of TMBQ to the known catalyst comprising free copper and halogen ions even in the reaction system where water is present and, furthermore, the drop in activity of the catalyst due to water is scarcely observed.

The copper halogeno complex constituting the catalyst of the present invention is a coordination compound of copper and halogen and is represented by the general formula (I):

$$M_l[Cu(II)_mX_n]_p \qquad (I)$$

wherein all the symbols are as defined above. This copper halogeno complex may or may not contain crystal water.

M in the formula (I) means an alkali metal or ammonium.

The alkali metal includes Li, K, Rb, and Cs. Preferred examples are Li, K, and Cs. Particularly preferred is Li.

Preferred examples of the halogen are Cl, Br, and I. Particularly preferred are Cl and Br.

Typical examples of the copper halogeno complex are shown below:

$Li[CuCl_3]\cdot 2H_2O$, $NH_4[CuCl_3]\cdot 2H_2O$, $(NH_4)_2[CuCl_4]\cdot 2H_2O$, $K[CuCl_3]$, $K_2[CuCl_4]\cdot 2H_2O$, $Cs[CuCl_3]\cdot 2H_2O$, $Cs_2[CuCl_4]\cdot 2H_2O$, $Cs_3[Cu_2Cl_7]\cdot 2H_2O$, $Li_2[CuBr_4]\cdot 6H_2O$, $K(CuBr_3]$, $(NH_4)_2[CuBr_4]\cdot 2H_2O$, $Cs_2[CuBr_4]$, and $Cs[CuBr_3]$.

These copper halogeno complexes can be prepared by known procedures such as those methods described in *Mellor's Comprehensive Treatment on Inorganic and Theoretical Chemistry*, Vol. III, pages 182—201 (Longman).

The copper halogeno complex thus prepared can be identified by known techniques such as by measuring its melting point. For example, the prepared copper chloride-lithium complex, $Li[CuCl_3]\cdot 2H_2O$, is red-brown in color and thus entirely different in appearance from cupric chloride, $CuCl_2\cdot 2H_2O$, which is a green crystal and the melting point of the complex is 130—135°C. The melting points of copper chloride-lithium complex, $Li[CuCl_3]\cdot 2H_2O$, and cupric chloride, $CuCl_2\cdot 2H_2O$, are 130°C and 110°C, respectively, according to *Mellor's Comprehensive Treatment on Inorganic and Theoretical Chemistry*, Vol. III, pages 184 and 169 (Longman).

Typical examples of the alkali metal halide which is used in combination with the copper halogeno complex are NaCl, LiCl, KCl, CsCl, NaBr, $NH_4Br$, KBr, CsBr, NaI, LiI, KI, and CsI. Of these compounds, LiCl is particularly preferred to use from viewpoints of yield and reaction rate.

The ratio of the copper halogeno complex to the alkali metal halide cannot be determined unconditionally because it varies with the type of the used copper halogeno complex or alkali metal halide, the solubility of the reaction mixture in water, and so forth. In general, the molar ratio of the alkali metal halide to the copper halogeno complex is usually 1:1 to 15:1, preferably 1:1 to 10:1, and especially preferably 2:1 to 5:1.

The concentration of the catalyst comprising the above copper halogeno complex and alkali metal halide in the aqueous phase of the reaction system is preferably 20 to 80 percent by weight (wt%), more preferably 20 to 70 wt%, and especially preferably 20 to 50 wt%.

In a basic embodiment of the present invention, the reaction is carried out in the presence of oxygen by adding a solution of TMP in a suitable alcohol solvent to a catalyst solution previously adjusted to a given composition and a given concentration at a given dropping rate.

In the above embodiment, the amounts of the copper halogeno complex and alkali metal halide used cannot be determined unconditionally because they vary depending on the type of the copper halogeno complex used, but it is preferred that the amount of the catalyst used is less than that in the batchwise reaction system. If the amount of the catalyst used is indicated in terms of the amount of the copper

halogeno complex, the amount of the copper halogeno complex used is 0.1 to 1 mole, preferably 0.1 to 0.5 mole, and especially preferably 0.1 to 0.3 mole per mole of the total amount of TMP continuously added to the reaction system.

The amount of the catalyst used in the above system is very small compared with that in the batchwise system. The amount of the catalyst used in the above system to provide the same yield of TMBQ as obtained under the optimum conditions of the batchwise system is about 1/4 of that in the batchwise system.

The alcohol that is used in the present ivnention is an aliphatic alcohol having from 5 to 10 carbon atoms. Preferred examples of such alcohols are n-amyl alcohol, isoamyl alcohol, n-hexyl alcohol, 2-ethylhexanol, n-heptyl alcohol, n-octyl alcohol, n-nonyl alcohol, and n-decyl alcohol.

In the present invention, since aliphatic alcohols having from 5 to 10 carbon atoms are used, the reaction system is completely heterogeneous. Astonishingly, however, the reaction is not adversely influenced at all and proceeds efficiently even in such a heterogeneous condition. Moreover, after the reaction is completed, an aqueous phase as the catalyst layer and an organic phase as an alcohol layer containing TMBQ can be easily separated by applying phase separation. For this reason, post-treatment for reuse of the catalyst and isolation of TMBQ can be very simplified.

The amount of the alcohol used in the present invention is not critical. It is necessary, however, for the alcohol to be used in such an amount as not to allow TMP to crystallize because the solution of TMP in the alcohol should be fed to the reaction system quantitatively and continuously. Taking into consideration the above factor and also the reaction efficiency, for example, the amount of the alcohol used can be determined appropriately. In general, the alcohol is used in such an amount that the concentration of TMP in the alcohol is from 5 to 50%, preferably from 10 to 50%, and especially preferably from 10 to 30%.

The reaction temperature varies with the type and amount of the copper halogeno complex or alkali metal halide, the concentration of the catalyst, and so forth. In general, the reaction temperature is preferably from 10 to 120°C, more preferably from 30 to 80°C, and most preferably from 40 to 80°C. The reaction temperature is not necessary to control so precisely. In the batchwise system, as the reaction scale is increased, it becomes more difficult to remove the reaction heat, and there is a danger of the reaction's running away. On the contrary, in the semi-batchwise reaction system of the present invention, the amount of the reaction heat per unit hour can be controlled by controlling the speed of feeding the starting material, TMP, and thus the reaction heat is easy to remove, which in turn makes it easy to control the reaction temperature.

In the preparation of TMBQ through oxidation of TMP with the catalyst of the present invention, comprising the copper halogeno complex and alkali metal halide, if the batchwise reaction system is employed, HMBP is formed in a relatively large amount. Thus the present invention is intended to depress the formation of HMBP, more increase the yield of TMBQ, and further to permit the use of oxygen gas of low concentration. In order to attain the above object, the relationship between the oxidation reaction rate and the feeding rate of TMP is very important. In a case that the feeding rate of TMP is made higher than a rate at which TMP is oxidized, the mode of the reaction becomes similar to the batchwise system. As a result, the amount of HMBP formed is increased and the advantage of the semi-batchwise reaction system is lost. In a case that the feeding rate of TMP is lower than the oxidation rate of TMP, the amount of HMBP formed is small and the features of the semi-batchwise reaction system are exhibited. However, if the feeding rate of TMP is decreased excessively, the reaction time is markedly lengthened and thus the space time yield is undesirably decreased. In a preferred embodiment of the present invention, therefore, the oxidation reaction rate is made equal to the feeding rate of TMP, or the feeding rate of TMP is made lower than the oxidation reaction rate. The oxidation reaction rate varies with the reaction temperature, the type and amount of the catalyst, and so forth, and the feeding rate of TMP can be determined experimentally. TMP is fed usually over 2 to 10 hours, preferably over 2 to 8 hours, and especially preferably over 3 to 5 hours.

In the reaction of the present invention, even after the introduction of TMP is completed, a small amount of 4-chlorotrimethylphenol (hereinafter abbreviated to "Cl-TMP"), an intermediate reaction product of TMBQ, remains in the reaction system, it is necessary for the oxidation reaction to be further continued. The time for this purpose cannot be determined unconditionally because it varies depending on other reaction conditions. In general, it is from 0.5 to 5 hours and preferably from 1 to 3 hours.

In the most basic embodiment of the semi-batchwise reaction of the present invention, a solution of TMP dissolved in a suitable solvent in a suitable concentration is supplied to a previously prepared aqueous catalyst solution at a suitable feeding rate in the presence of an oxygen-containing gas having an oxygen concentration of at least 20%. In this case, however, there is an induction period at the initial stage of the reaction although this is not so significant when 100% oxygen is used. For this reason, the reaction proceeds as nearly batchwise at the initial stage thereof although for a relatively short period of time, thereby increasing the amount of HMBP formed and decreasing the TMBQ yield. This tendency becomes more marked as the concentration of oxygen in the gas is more reduced and, furthermore, the oxidation reaction rate drops. Therefore, in a case that the concentration of oxygen in the gas used is small, it is necessary to decrease the feeding rate of TMP, thereby depressing the formation of HMBP, so as to increase the TMBQ yield. This, however, is undesirable because the reaction time is excessively increased.

In the process of the present invention, therefore, the same alcohol solvent as used in the preparation

of the TMP solution, cupric hydroxide, cuprous chloride, or a mixture thereof is previously added to the reaction system, so that the induction period of the reaction can be avoided and the oxidation reaction can be carried out satisfactorily even if an oxygen-containing gas having an oxygen concentration of about 20%, i.e., air, is used. In a case that the alcohol solvent is added to the reaction system prior to the oxidation reaction of TMP, the amount of the alcohol added is from 0.05 to 1.3 times, preferably from 0.05 to 0.65 time, and especially preferably from 0.13 to 0.26 time the weight of the alcohol solvent contained in the whole TMP solution. The amount of cupric hydroxide or cuprous chloride used is limited by its solubility in the reaction system. In general, the amount of cupric hydroxide or cuprous chloride used is from 0.01 to 0.05 mole per mole of the copper halogeno complex.

The induction period at the initial stage of the reaction can be eliminated, as described above, by adding the alcohol solvent, cupric hydroxide, cuprous chloride, or a mixture thereof. In a case that cupric hydroxide or cuprous chloride is used, the rate of oxidation into TMBQ of Cl-TMP remaining in the reaction system after the dropwise addition of the starting material is increased and, as a result, the total reaction time is desirably shortened.

The starting material, TMP, used in the present invention is not limited by its method of preparation. For example, TMPs prepared by alkylation of phenols (e.g., m-cresol, 2,3-xylenol, and 2,5-xylenol), transalkylation of polymethylphenol, alkali fusion of 2,3,6-trimethylbenzenesulfonic acid, oxidation of 2,3,6-tri-methylcumene, distillation of a tar fraction, and by isolation from phenols can be used.

The term "oxygen" as used herein includes both pure oxygen and an oxygen-containing gas. The oxygen-containing gas includes an oxygen enriched air, air, and oxygen diluted with inert gas. Inert gases which can be used for this purpose include nitrogen, helium, and argon. The concentration of oxygen in the gas is preferably from 20 to 100%.

The pressure as an oxygen partial pressure is from 0.049 to 49 bar [0.05 to 50 kilograms per square centimeter ($kg/cm^2$)] (absolute pressure), preferably from 0.098 to 19.6 bar (0.1 to 20 $kg/cm^2$), and more preferably from 0.196 to 9.8 bar (0.2 to 10 $kg/cm^2$). Most preferred is a pressure ranging between 0.294 to 0.98 bar (0.3 and 1 $kg/cm^2$).

The present invention is basically carried out by contacting TMP with oxygen in water and alcohol in the presence of the copper halogeno complex and alkali metal halide. In the most preferred embodiment, the reaction is carried out in a reactor with a stirrer, because good contact of gas with liquid is attained efficiently.

The reaction of the present invention is carried out by two methods. One of the methods is to pass an oxygen gas through the reactor, and the other method is a gas circulation method in which a given pressure is maintained by compensating for absorbed oxygen.

In accordance with the present invention, a mixture of the copper halogeno complex and alkali metal halide is used as a catalyst, an aliphatic alcohol having from 5 to 10 carbon atoms, cupric hydroxide, cuprous chloride or a mixture thereof is previously added to the aqueous catalyst solution, and the reaction is carried out semi-batchwise. Thus, compared with the batchwise reaction system, the amount of the catalyst used can be greatly reduced, the yield of TMBQ can be increased, and furthermore the control of the reaction temperature can be facilitated.

In the present invention, the product, TMBQ, can be easily separated. That is, since an aliphatic alcohol having from 5 to 10 carbon atoms is used, the aqueous layer and organic layer are always separated from each other. Thus, TMBQ can be obtained by distilling away the solvent from the organic layer. The aqueous layer can be used repeatedly as a catalyst solution as it is or, if necessary, after concentration or purification. Therefore, the process of the present invention is excellent from an industrial standpoint.

The organic layer thus obtained does not almost contain by-products. Thus, when it is washed with water and then reduced as such, a precursor for vitamin E, 2,3,5-trimethylhydroquinone, can be obtained in a high purity.

The present invention is described in greater detail with reference to the following examples and comparative examples. In the examples and comparative examples, all conversions and yields are expressed in terms of mole.

## Comparative Example 1

A copper halogeno complex, $Li[CuCl_3]\cdot 2H_2O$, lithium chloride, and water were placed in a 1-liter four-necked flask to prepare a catalyst solution. After replacement of the atmosphere in the flask with oxygen, the catalyst solution was heated from the outside and vigorously stirred at 950 revolutions per minute (rpm). When the inner temperature reached 60°C, a 30% hexanol solution of 2,3,6-TMP was added dropwise at a rate of 38.3 grams per hour (g/hr) by the use of a constant volume pump. 100% Oxygen was successively supplied from a gas holder, and the amount of oxygen consumed was measured with a wet-type gas flow meter. When 115 g of the 30% hexanol solution of TMP (0.26 mole of TMP) was added (the time required: 3 hours), its introduction was stopped. Subsequently, the resulting mixture was stirred for 1 hour at the same temperature. In this example, about 5 minutes of an induction period was observed. After the reaction, an aqueous layer and an organic layer were separated from each other. After the separation of the layers, the alcohol layer was analyzed by gas chromatography. The results are shown in Table 1.

Table 1

| Run No. | TMP/Complex /LiCl (molar ratio) | Total Concentration of Complex and LiCl (wt%) | Conversion of TMP (%) | Yield of TMBQ (%) | Yield of HMBP (%) | Yield of 4-Cl-TMP (%) |
|---|---|---|---|---|---|---|
| Comparative Example 1 | 1/0.25/0.75 | 41 | 100 | 96.2 | 2.3 | 0 |

Examples 1 to 5

The procedure of Comparative Example 1 was repeated wherein 20 g of hexanol, 0.2 g of cupric chloride, 0.3 g of cuprous chloride, or a mixture thereof was previously added to the catalyst solution. The induction period observed at the initial stage of the reaction in Comparative Example 1 was not observed in these examples. The results are shown in Table 2.

Table 2

| Run No. | Compound added previously | Conversion of TMP (%) | Yield of TMBQ (%) | Yield of HMBP (%) |
|---|---|---|---|---|
| Example 1 | Hexanol | 100 | 97.2 | 1.8 |
| Example 2 | Cupric hydroxide | 100 | 97.6 | 1.1 |
| Example 3 | Cuprous chloride | 100 | 97.3 | 1.1 |
| Example 4 | Hexanol + Cupric hydroxide | 100 | 97.5 | 1.2 |
| Example 5 | Hexanol + Cuprous chloride | 100 | 97.4 | 1.1 |

Examples 6 to 13 and
Comparative Examples 2 to 3

The reaction was carried out using a gas having an oxygen concentration of 20% (air) and a gas having an oxygen concentration of 40% (air + 100% oxygen) in the same manner as in Examples 1 to 5 except that the dropping rate of the TMP solution was 19.2 g/hr (the dropping period of time was 6 hours) in Comparative Example 2 and 28.75 g/hr (the dropping period of time was 4 hours) in the other examples. After the dropwise addition of the TMP solution was completed, the reaction mixture was stirred for 1.5 hours, and the products were analyzed. The results are shown in Table 3.

Table 3

| Run No. | Oxygen Concentration (%) | Compound added previously | Induction Period (min) | Conversion of TMP (%) | Yield of TMBQ (%) | Yield of HMBP (%) | Yield of 4-Cl-TMP (%) |
|---|---|---|---|---|---|---|---|
| Comparative Example 2 | 20 | - | 30 | 100 | 90 | 6.5 | 2.0 |
| Example 6 | 20 | Hexanol | 5 | 100 | 95 | 3.5 | 0.5 |
| Example 7 | 20 | Cupric hydroxide | 5 | 100 | 94.5 | 3.5 | 0.3 |
| Example 8 | 20 | Cuprous chloride | 5 | 100 | 94.7 | 3.0 | 0.6 |
| Example 9 | 20 | Hexanol + Cupric hydroxide | 0 | 100 | 96.8 | 2.1 | 0 |
| Comparative Example 3 | 40 | | 10 | 100 | 91.5 | 6.5 | 0 |
| Example 10 | 40 | Hexanol | 0 | 100 | 97.2 | 1.9 | 0 |
| Example 11 | 40 | Cupric hydroxide | 0 | 100 | 97.4 | 1.0 | 0 |
| Example 12 | 40 | Cuprous chloride | 0 | 100 | 97.0 | 1.0 | 0 |
| Example 13 | 40 | Hexanol + Cupric hydroxide | 0 | 100 | 97.1 | 1.3 | 0 |

Examples 14 and 15

The procedure of Example 1 was repeated wherein the dropping rate of the TMP solution was changed to 57.5 g/hr and 23 g/hr (the dropping period of time was 2 hours and 5 hours). The results are shown in Table 4.

Table 4

| Run No. | Dropping Period of Time (hr) | Conversion of TMP (%) | Yield of TMBQ (%) | Yield of HMBP (%) | Yield of 4-Cl-TMP (%) |
|---|---|---|---|---|---|
| Example 14 | 2.0 | 100 | 95.8 | 3.0 | 0.6 |
| Example 15 | 5.0 | 100 | 96.9 | 0.5 | 0 |

Examples 16 to 20

The procedure of Example 1 was repeated wherein alcohols described in Table 5 were used as the reaction solvent. The results are shown in Table 5.

Table 5

| Run No. | Alcohol | Conversion of TMP (%) | Yield of TMBQ (%) | Yield of HMBP (%) | Yield of 4-Cl-TMP (%) |
|---|---|---|---|---|---|
| Example 16 | n-Amyl alcohol | 100 | 97.1 | 2.0 | 0 |
| Example 17 | n-Heptyl alcohol | 100 | 97.0 | 1.8 | 0 |
| Example 18 | n-Octyl alcohol | 100 | 97.3 | 2.3 | 0 |
| Example 19 | n-Nonyl alcohol | 100 | 97.2 | 2.0 | 0 |
| Example 20 | n-Decyl alcohol | 100 | 97.0 | 2.0 | 0 |

Examples 21 and 22

The procedure of Example 1 was repeated wherein the amount of the catalyst used was changed. The results are shown in Table 6.

Table 6

| Run No. | TMP/Complex/LiCl (molar ratio)* | Conversion of TMP (%) | Yield of TMBQ (%) | Yield of HMBP (%) | Yield of 4-Cl-TMP (%) |
|---|---|---|---|---|---|
| Example 21 | 1/1/3 | 100 | 95.0 | 2.1 | 1.1 |
| Example 22 | 1/0.5/1.5 | 100 | 96.3 | 2.0 | 0.8 |

Note: *Catalyst concentration in the aqueous layer; 41 wt%.

Example 23

The procedure of Example 1 was repeated wherein the catalyst concentration was increased. The results are shown in Table 7.

Table 7

| Run No. | Catalyst Concentration (%) | Conversion of TMP (%) | Yield of TMBQ (%) | Yield of HMBP (%) | Yield of 4-Cl-TMP (%) |
|---|---|---|---|---|---|
| Example 23 | 55 | 100 | 96.2 | 1.8 | 1.1 |

Examples 24 and 25

The procedure of Example 1 was repeated wherein the amount of lithium chloride added was changed, and the catalyst concentration was adjusted to 41%. The results are shown in Table 8.

Table 8

| Run No. | Amount of LiCl added (g) | TMP/Complex/ LiCl (molar ratio) | Conversion of TMP (%) | Yield of TMBQ (%) | Yield of HMBP (%) | Yield of 4-Cl-TMP (%) |
|---|---|---|---|---|---|---|
| Example 24 | 16.6 | 1/0.25/2 | 100 | 94.1 | 2.0 | 3.0 |
| Example 25 | 4.2 | 1/0.25/0.5 | 100 | 93.5 | 5.0 | 0.8 |

Comparative Example 5

In this example, the reaction was carried out batchwise.

Given amounts of a copper halogeno complex, $Li[CuCl_3]\cdot 2H_2O$, lithium chloride, and water were placed in a 1-liter four-necked flask to prepare a catalyst solution. Then, 115 g of a 30% hexanol solution of 2,3,6-TMP was added to the flask and, after replacement of the atmosphere in the flask with oxygen, the resulting mixture was heated from the outside. When the inner temperature reached 60°C, the mixture was vigorously stirred at 950 rpm. Oxygen was successively supplied from a gas holder, and the amount of oxygen consumed was measured by the use of a wet-type gas flow meter. After gas absorption stopped, stirring was further continued for 1 hour at the same temperature. When the reaction was completed, the reaction mixture was separated into aqueous and alcohol layers. The alcohol layer was separated from the aqueous layer and analyzed by gas chromatography. The results are shown in Table 9.

Table 9

| Run No. | TMP/Complex/ LiCl (molar ratio) | Conversion of TMP (%) | Yield of TMBQ (%) | Yield of HMBP (%) | Yield of 4-Cl-TMP (%) |
|---|---|---|---|---|---|
| Comparative Example 5 | 1/0.25/0.75 | 100 | 87.5 | 11.0 | 0.2 |

Comparative Examples 6 and 7

The procedure of Comparative Example 5 was repeated wherein the reaction was conducted in the presence of cuprous chloride or cupric hydroxide. The results are shown in Table 10.

Table 10

| Run No. | Compound added previously | Conversion of TMP (%) | Yield of TMBQ (%) | Yield of HMBP (%) | Yield of 4-Cl-TMP (%) |
|---|---|---|---|---|---|
| Comparative Example 6 | Cupric hydroxide 0.2 g | 100 | 86.7 | 10.8 | 0 |
| Comparative Example 7 | Cuprous chloride 0.15 g | 100 | 87.3 | 11.3 | 0 |

Comparative Examples 8 and 9

The procedure of Comparative Example 6 was repeated wherein a gas having an oxygen concentration of 20% or 40% was used. The results are shown in Table 11.

Table 11

| Run No. | Oxygen Concentration (%) | Conversion of TMP (%) | Yield of TMBQ (%) | Yield of HMBP (%) | Yield of 4-Cl-TMP (%) |
|---|---|---|---|---|---|
| Comparative Example 8 | 20 | 100 | 85.1 | 12.3 | 0.5 |
| Comparative Example 9 | 40 | 100 | 86.0 | 11.0 | 0 |

Example 26, and Comparative Example 10

In Example 26, the procedure of Example 1 was repeated wherein $Li_2[CuBr_4]\cdot 6H_2O$ was used as the copper halogeno complex, and LiCl as the alkali metal halide.

In Comparative Example 10, the procedure of Comparative Example 5 was repeated wherein $Li_2[CuBr_4]\cdot 6H_2O$ was used as the copper halogeno complex, and LiCl as the alkali metal halide.

The results are shown in Table 12.

Table 12

| Run No. | TMP/Complex/LiCl (molar ratio) | Conversion of TMP (%) | Yield of TMBQ (%) | Yield of HMBP (%) | Yield of 4-Cl-TMP (%) |
|---|---|---|---|---|---|
| Example 26 | 1/0.3/1 | 100 | 90.1 | 6.5 | 0 |
| Comparative Example 10 | 1/4/1 | 100 | 87.9 | 10.1 | 0 |

Examples 27 to 32

The procedure of Example 1 was repeated wherein the copper halogeno complexes and alkali metal halides shown in Table 13 were used. The results are also shown in Table 13.

Table 13

| Run No. | Complex* | Alkali metal halide | Conversion of TMP (%) | Yield of TMBQ (%) | Yield of HMBP (%) | Yield of 4-Cl-TMP (%) |
|---|---|---|---|---|---|---|
| Example 27 | A | LiCl | 100 | 96.9 | 1.9 | 0 |
| Example 28 | B | " | 100 | 97.0 | 2.0 | 0 |
| Example 29 | C | " | 100 | 97.1 | 2.1 | 0 |
| Example 30 | D | " | 100 | 97.0 | 1.8 | 0 |
| Example 31 | E | CsCl | 100 | 96.9 | 2.3 | 0 |
| Example 32 | F | " | 100 | 96.8 | 2.4 | 0 |

Note: *Complex A; $(NH_4)[CuCl_3]\cdot 2H_2O$
" B; $K[CuCl_3]\cdot 2H_2O$
" C; $K_2[CuCl_4]\cdot 2H_2O$
" D; $Cs[CuCl_3]$
" E; $Cs_2[CuCl_4]\cdot 2H_2O$
" F; $Cs_2[CuBr_4]$

**Claims**

1. A process for preparing 2,3,5-trimethylbenzoquinone by contacting 2,3,6-trimethylphenol with oxygen or oxygen-containing gas having an oxygen concentration of at least 20% in an aqueous solution of a catalyst comprising a copper halogeno complex represented by the general formula (I) and an alkali metal halide,

$$M_l[Cu(II)_mX_n]_p \quad (I)$$

wherein M is an alkali metal belonging to IA of the Periodic Table or ammonium, Cu(II) is a divalent copper, X is a halogen atom, l is an integer of 1 to 3, m is 1 or 2, n is an integer of 3 to 8, and p is 1 or 2, and l + 2 mp = np; said contact reaction of 2,3,6-trimethylphenol with the oxygen or the oxygen-containing gas being carried out semi-batchwise while continuously adding a solution of 2,3,6-trimethylphenol in an aliphatic alcohol having from 5 to 10 carbon atoms to the aqueous solution of the catalyst to which an aliphatic alcohol having from 5 to 10 carbon atoms, cupric hydroxide, cuprous chloride or a mixture thereof is previously added.

2. The process as claimed in Claim 1, wherein the solution of 2,3,6-trimethylphenol is added over a period of at least 2 hours.

3. The process as claimed in Claim 1, wherein the aliphatic alcohol having from 5 to 10 carbon atoms is selected from the group consisting of n-amyl alcohol, n-hexyl alcohol, 2-ethyl hexanol, n-heptyl alcohol, n-octyl alcohol, n-nonyl alcohol, and n-decyl alcohol.

4. The process as claimed in Claim 1, wherein the concentration of 2,3,6-trimethylphenol in the aliphatic alcohol having from 5 to 10 carbon atoms is from 5 to 50 percent by weight.

5. The process as claimed in Claim 1, wherein the concentration of 2,3,6-trimethylphenol in the aliphatic alcohol having from 5 to 10 carbon atoms is from 10 to 50 percent by weight.

6. The process as claimed in Claim 1, wherein the weight ratio of the amount of the aliphatic alcohol having from 5 to 10 carbon atoms as previously added to the aqueous solution of the castalyst to the total amount of the alcohol being added is from 0.05:1 to 1.3:1.

7. The process as claimed in Claim 1, wherein the amount of the cupric hydroxide previously added to the aqueous solution of the catalyst is from 0.01 to 0.05 mole per mole of the copper halogeno complex used.

8. The process as claimed in Claim 1, wherein the amount of cuprous chloride previously added to the aqueous solution of the catalyst is from 0.01 to 0.05 mole per mole of the copper halogeno complex used.

9. The process as claimed in Claim 1, wherein the copper halogeno complex is selected from the group consisting of $Li[CuCl_3]\cdot 2H_2O$, $NH_4[CuCl_3]\cdot 2H_2O$, $(NH_4)_2[CuCl_4]\cdot 2H_2O$, $K[CuCl_3]$, $K_2[CuCl_4]\cdot 2H_2O$, $Cs[CuCl_3]\cdot 2H_2O$, $Cs_2[CuCl_4]\cdot 2H_2O$, $Cs_3[Cu_2Cl_7]\cdot 2H_2O$, $Li_2[CuBr_4]\cdot 6H_2O$, $K(CuBr_3]$, $(NH_4)_2[CuBr_4]\cdot 2H_2O$, $Cs_2[CuBr_4]$, and $Cs[CuBr_3]$.

10. The process as claimed in Claim 1, wherein the copper halogeno complex may or may not contain crystal water.

11. The process as claimed in Claim 1, wherein the alkali metal halide is at least one selected from the group consisting of NaCl, LiCl, KCl, CsCl, NaBr, $NH_4Br$, KBr, CsBr, NaI, LiI, KI, and CsI.

12. The process as claimed in Claim 1, wherein the molar ratio of the copper halogeno complex to the alkali metal halide is from 1:1 to 1:15.

13. The process as claimed in Claim 1, wherein the molar ratio of the copper halogeno complex to the alkali metal halide is from 1:1 to 1:10.

14. The process as claimed in Claim 1, wherein the total concentration of the copper halogeno complex and alkali metal halide in the aqueous layer of the reaction system is from 20 to 80 percent by weight.

15. The process as claimed in Claim 12, wherein the total concentration of the copper halogeno complex and alkali metal halide in the aqueous layer of the reaction system is from 20 to 70 percent by weight.

16. The process as claimed in Claim 1, wherein the molar ratio of the copper halogeno complex to 2,3,6-trimethylphenol is from 1:10 to 10:10.

17. The process as claimed in Claim 1, wherein the molar ratio of the copper halogeno complex to 2,3,6-trimethylphenol is from 1:10 to 5:10.

18. The process as claimed in Claim 1, wherein the oxygen concentration of the gas used is from 20 to 100 percent.

**Patentansprüche**

1. Verfahren zur Herstellung von 2,3,5-Trimethylbenzochinon durch in Kontakt bringen von 2,3,6-Trimethylphenol mit Sauerstoff oder Sauerstoff enthaltendem Gas mit einer Sauerstoffkonzentration von mindestens 20% in einer wäßrigen Lösung eines Katalysators, enthaltend einen Kupferhalogenokomplex dargestellt durch die allgemeine Formel (I) und einem Alkalimetallhalogenid,

$$M_l[Cu(II)_mX_n]_p \quad (I)$$

worin M ein Alkalimetall angehörend der IA des Periodensystems oder Ammonium ist, Cu(II) ein zweiwertiges Kupfer ist, X ein Halogenatom ist, l eine ganze Zahl von 1 bis 3 ist, m 1 oder 2 ist, n eine ganze Zahl von 3 bis 8 ist, p 1 oder 2 ist und l + 2 mp = np ist; wobei die Kontaktreaction von 2,3,6-Trimethylphenol mit dem Sauerstoff oder dem sauerstoffhaltigen Gas semi-satzweise durchgeführt wird, während kontinuierlicher Zugabe einer Lösung von 2,3,6-Trimethylphenol in einem aliphatischen Alkohol mit 5 bis 10 Kohlenstoffatomen zur wäßrigen Lösung des Katalysators, zu der ein aliphatischer Alkohol mit 5 bis 10 Kohlenstoffatomen, Kupferhydroxid, Kupfer-(I)-chlorid oder ein Gemisch davon vorher hinzugefügt wurde.

2. Verfahren nach Anspruch 1, worin die Lösung von 2,3,6-Trimethylphenol über eine Zeitdauer von mindestens zwei Stunden hinzugefügt wird.

3. Verfahren nach Anspruch 1, worin der aliphatische Alkohol mit 5 bis 10 Kohlenstoffatomen, ausgewählt ist aus der Gruppe, bestehend aus n-Amylalkohol, n-Hexylalkohol, 2-Ethylhexanol, n-Heptylalkohol, n-Octylalkohol, n-Nonylalkohol und n-Decylalkohol.

4. Verfahren nach Anspruch 1, worin die Konzentration des 2,3,6-Trimethylphenols im aliphatischen Alkohol mit 5 bis 10 Kohlenstoffatomen von 5 bis 50 Gew.-% ist.

5. Verfahren nach Anspruch 1, worin die Konzentration des 2,3,6-Trimethylphenols im aliphatischen Alkohol mit 5 bis 10 Kohlenstoffatomen von 10 bis 50 Gew.-% ist.

6. Verfahren nach Anspruch 1, worin das Gewichtsverhältnis der Menge des vorher zur wäßrigen Lösung des Katalysators zugegebenen aliphatischen Alkohols mit 5 bis 10 Kohlenstoffatomen zur Gesamtmenge des hinzugefügten Alkhols von 0,05:1 bis 1,3:1 ist.

7. Verfahren nach Anspruch 1, worin die Menge des vorher zur wäßrigen Lösung des Katalysators zugegebenen Kupferhydroxyds von 0,01 bis 0,05 Mol pro Mol des verwendeten Kupferhalogenokomplexes ist.

8. Verfahren nach Anspruch 1, worin die Menge des vorher zur wäßrigen Lösung des Katalysators hinzugefügten Kupfer-(I)-chlorids von 0,01 bis 0,05 Mol pro Mol der verwendeten Kupferhalogenokomplexes ist.

9. Verfahren nach Anspruch 1, worin der Kupferhalogenokomplex ausgewählt ist aus der Gruppe, bestehend aus $Li[CuCl_3]\cdot 2H_2O$, $NH_4[CuCl_3]\cdot 2H_2O$, $(NH_4)_2[CuCl_4]\cdot 2H_2O$, $K[CuCl_3]$, $K_2[CuCl_4]\cdot 2H_2O$, $Cs[CuCl_3]\cdot 2H_2O$, $Cs_2[CuCl_4]\cdot 2H_2O$, $Cs_3[Cu_2Cl_7]\cdot 2H_2O$, $Li_2[CuBr_4]\cdot 6H_2O$, $K(CuBr_3]$, $(NH_4)_2[CuBr_4]\cdot 2H_2O$, $Cs_2[CuBr_4]$ und $Cs[CuBr_3]$.

10. Verfahren nach Anspruch 1, worin der Kupferhalogenokomplex Kristallwasser enthalten kann oder nicht.

11. Verfahren nach Anspruch 1, worin das Alkalimetallhalogenid mindestens eines ist ausgewählt aus der Gruppe, bestehend aus NaCl, LiCl, KCl, CsCl, NaBr, $NH_4Br$, KBr, CsBr, NaI, LiI, KI und CsI.

12. Verfahren nach Anspruch 1, worin das molare Verhältnis der Kupferhalogenokomplexes zum Alkalimetallhalogenid von 1:1 bis 1:15 ist.

13. Verfahren nach Anspruch 1, worin das molare Verhältnis des Kupferhalogenokomplexes zum Alkalimetallhalogenid von 1:1 bis 1:10 ist.

14. Verfahren nach Anspruch 1, worin die Gesamtkonzentration des Kupferhalogenokomplexes und Alkalimetallhalogenids in der wäßrigen Phase des Reaktionssystems von 20 bis 80 Gew.-% ist.

15. Verfahren nach Anspruch 12, worin die Gesamtkonzentration des Kupferhalogenokomplexes und Metallhalogenids in der wäßrigen Phase des Reaktionssystems von 20 bis 70 Gew.-% ist.

16. Verfahren nach Anspruch 1, worin das molare Verhältnis des Kupferhalogenokomplexes zu 2,3,6-Trimethylphenol von 1:10 bis 10:10 ist.

17. Verfahren nach Anspruch 1, worin das molare Verhältnis des Kupferhalogenokomplexes zu 2,3,6-Trimethylphenol von 1:10 bis 5:10 ist.

18. Verfahren nach Anspruch 1, worin die Sauerstoffkonzentration des verwendeten Gases von 20 bis 100% ist.

**Revendications**

1. Un procédé de préparation de la 2,3,5-triméthylbenzoquinone par contact du 2,3,6-triméthylphénol avec de l'oxygène ou un gaz contenant de l'oxygène ayant une concentration en oxygène d'au moins 20% dans une solution aqueuse d'un catalyseur comprenant un complexe halogéné du cuivre représenté par la formule générale (I) et un halogénure de métal alcalin,

$$M_l[Cu(II)_mX_n]_p \quad (I)$$

où M est un métal alcalin appartenant au groupe IA du Tableau Périodique ou l'ammonium, Cu(II) est un cuivre divalent, X est un atome d'halogène, l est un entier de 1 à 3, m est 1 ou 2, n est un entier de 3 à 8, p est 1 ou 2, et 1 + 2 mp = np; ladite réaction de contact du 2,3,6-triméthylphénol avec l'oxygène ou le gaz renfermant de l'oxygène étant réalisée en semi-discontinu tout en ajoutant continuellement une solution de 2,3,6-triméthylphénol dans un alcool aliphatique ayant de 5 à 10 atomes de carbone à la solution aqueuse du catalyseur à laquelle on a préalablement ajouté un alcool aliphatique de 5 à 10 atomes de carbone, de l'hydroxyde cuivrique, du chlorure cuivreux ou un mélange de ceux-ci.

2. Le procédé tel que revendiqué dans la Revendication 1, dans lequel la solution de 2,3,6-triméthylphénol est ajoutée pendant une période d'au moins 2 heures.

3. Le procédé tel que revendiqué dans la Revendication 1, où l'alcool aliphatique ayant de 5 à 10 atomes de carbone est choisi dans le groupe consistant en alcool n-amylique, alcool n-hexylique, 2-éthylhexanol, alcool n-heptylique, alcool n-octylique, alcool n-nonylique, et alcool n-décylique.

4. Le procédé tel que revendiqué dans la Revendication 1, dans lequel la concentration en 2,3,6-triméthylphénol dans l'alcool aliphatique ayant de 5 à 10 atomes de carbone est de 5 à 50% en poids.

5. Le procédé tel que revendiqué dans la Revendication 1, dans lequel la concentration en 2,3,6-triméthylphénol dans l'alcool aliphatique ayant de 5 à 10 atomes de carbone est de 10 à 50% en poids.

6. Le procédé tel que revendiqué dans la Revendication 1, dans lequel le rapport pondéral de la quantité d'alcool aliphatique ayant de 5 à 10 atomes de carbone ajouté préalablement à la solution aqueuse de catalyseur à la quantité totale de l'alcool ajouté est de 0,05:1 à 1,3:1.

7. Le procédé tel que revendiqué dans la Revendication 1, dans lequel la quantité d'hydroxyde cuivrique préalablement ajouté à la solution aqueuse du catalyseur est de 0,01 à 0,05 mole par mole du complexe halogéné du cuivre utilisé.

8. Le procédé tel que revendiqué dans la Revendication 1, dans lequel la quantité de chlorure cuivreux préalablement ajouté à la solution aqueuse de catalyseur est de 0,01 à 0,05 mole par mole du complexe halogéné du cuivre.

9. Le procédé tel que revendiqué dans la Revendication 1, dans lequel le complexe halogéné du cuivre est choisi dans le groupe comprenant:
$Li[CuCl_3]\cdot 2H_2O$, $NH_4[CuCl_3]\cdot 2H_2O$, $(NH_4)_2[CuCl_4]\cdot 2H_2O$, $K[CuCl_3]$, $K_2[CuCl_4]\cdot 2H_2O$, $Cs[CuCl_3]\cdot 2H_2O$, $Cs_2[CuCl_4]\cdot 2H_2O$, $Cs_3[Cu_2Cl_7]\cdot 2H_2O$, $Li_2[CuBr_4]\cdot 6H_2O$, $K(CuBr_3]$, $(NH_4)_2[CuBr_4]\cdot 2H_2O$, $Cs_2[CuBr_4]$, et $Cs[CuBr_3]$.

10. Le procédé tel que revendiqué dans la Revendication 1, dans lequel le complexe halogéné du cuivre peut ou non renfermer de l'eau de constitution.

11. Le procédé tel que revendiqué dans la Revendication 1, dans lequel l'halogénure de métal alcalin est au moins l'un choisi dans le groupe consistant en NaCl, LiCl, KCl, CsCl, NaBr, $NH_4Br$, KBr, CsBr, NaI, LiI, KI, et CsI.

12. Le procédé tel que revendiqué dans la Revendication 1, dans lequel le rapport molaire du complexe halogéné du cuivre à l'halogénure de métal alcalin est de 1:1 à 1:15.

13. Le procédé tel que revendiqué dans la Revendication 1, dans lequel le rapport molaire du complexe halogéné du cuivre à l'halogénure de métal alcalin est de 1:1 à 1:10.

14. Le procédé tel que revendiqué dans la Revendication 1, dans lequel la concentration totale du complexe halogéné du cuivre et de l'halogénure de métal alcalin dans la couche aqueuse du système réactionnel est de 20 à 80% en poids.

15. Le procédé tel que revendiqué dans la Revendication 12, dans lequel la concentration totale du complexe halogéné du cuivre et de l'halogénure de métal alcalin dans la couche aqueuse du système réactionnel est de 20 à 70% en poids.

16. Le procédé tel que revendiqué dans la Revendication 1, dans lequel le rapport molaire du complexe halogéné du cuivre au 2,3,6-triméthylphénol est de 1:10 à 10:10.

17. Le procédé tel que revendiqué dans la Revendication 1, dans lequel le rapport molaire du complexe halogéné du cuivre au 2,3,6-triméthylphénol est de 1:10 à 5:10.

18. Le procédé tel que revendiqué dans la Revendication 1, dans lequel la concentration en oxygène du gaz utilisé est de 20 à 100%.